# EUROPEAN PATENT APPLICATION

(11) **EP 2 078 517 A2**
(43) Date of publication of application: **15.07.2009**
(21) Application number: 09000170.2
(22) Date of filing: 08.01.2009
(51) Int. Cl.: A61F 13/02

(54) **Method of using adhesive patch**

(30) Priority: 10.01.2008 JP 2008003166
(71) Applicant: Nitto Denko Corporation, Ibaraki-shi, Osaka 567-8680 (JP)
(72) Inventor: Harima, Jun c/o Nitto Denko Corporation, Osaka 567-8680 (JP)
(74) Representative: Grünecker, Kinkeldey, Stockmair & Schwanhäusser Anwaltssozietät

(57) **Abstract**

A subject for the invention is to provide a method of using an adhesive patch employing a grooved liner, by which the liner can be easily peeled off. The invention provides a method of using an adhesive patch which includes a backing, a pressure-sensitive adhesive layer formed on one surface of the backing, and a liner having a thickness T and laminated on the pressure-sensitive adhesive layer, in which the liner has a groove formed from the surface opposite to the surface on which the pressure-sensitive adhesive layer is laminated and having a depth of from T/2 to less than T, and the groove has a plane shape which enables the liner to be divided into two or more liner pieces by the groove,

## Description

### FIELD OF THE INVENTION

The present invention relates to a method of using an adhesive patch including a backing, a pressure-sensitive adhesive layer formed on one surface of the backing, and a liner laminated on the pressure-sensitive adhesive layer.

### BACKGROUND OF THE INVENTION

Various adhesive patches have been developed in recent years. These adhesive patches are used in various applications. In particular, adhesive patches for the protection of a wound and for the continuous percutaneous administration of a drug are highly excellent. Such an adhesive patch is constituted of a backing, such as a fabric or plastic film, a pressure-sensitive adhesive layer superposed on the backing, and a liner superposed on the pressure-sensitive adhesive layer. Such a liner not only protects the application side of the pressure-sensitive adhesive layer but also produces the following effect. Namely, when the liner is used in an adhesive patch employing a flexible backing, it improves the self-holding properties of the adhesive patch to thereby improve the handleability of the adhesive patch. When such an adhesive patch is used, the user peels off the liner from the pressure-sensitive adhesive layer and applies the exposed application side of the pressure-sensitive adhesive layer to a given skin part. The liner is hence desired to be easily peeled from the pressure-sensitive adhesive layer.

JP-A-2003-033389 discloses an adhesive patch which includes a backing, a pressure-sensitive adhesive layer formed on one side of the backing, and a liner superposed on the pressure-sensitive adhesive layer and having a thickness T, in which the liner has a groove formed from the surface opposite to the surface on which the pressure-sensitive adhesive layer is laminated. In this adhesive patch, the groove has a depth smaller than the thickness T of the liner and does not reach the pressure-sensitive adhesive layer. Namely, the liner is not separated into two or more liner pieces until just before use. The document includes a statement concerning this example to the effect that the groove is regulated so as to have such a depth that the groove does not reach the pressure-sensitive adhesive layer and an exceedingly thin liner-connecting part is present at the bottom of the groove to cover the pressure-sensitive adhesive layer and prevent the pressure-sensitive adhesive layer from oozing out through the groove and thereby impairing handleability or adversely influencing stability (see, paragraph 0028). With respect to a method of using this adhesive patch, the document includes a statement to the effect that the depth of the groove is desirably regulated so as to be 14T/15 or larger to thereby enable the liner to be easily tom into two liner pieces.

However, this document does not disclose to bend the adhesive patch along the groove. It is a matter of course that the document does not suggest that the adhesive patch is bent along the groove to thereby break the liner-connecting part located at the groove bottom.

As a matter of fact, the document includes a statement to the effect that in another example having such a connecting part at the groove bottom, the liner can be easily "torn" to two liner pieces (paragraph 0038). For conducting such an operation, it is necessary to apply a sufficient tensile force to the groove-bottom connecting part of the liner using both hands, i.e., while using one hand to tightly hold one end of the adhesive patch and using the other hand to tightly hold the other end of the patch. It is hence suggested that it is not easy to peel off such a release liner before using the adhesive patch.

On the other hand, such an adhesive patch in which the liner is not separated into two or more liner pieces until just before use is superior in the following points: (i) since the pressure-sensitive adhesive layer is not exposed to the environment due to the connecting part located at the groove bottom, a component of the pressure-sensitive adhesive layer is inhibited from oozing out through the groove during patch storage and adhering to the inner surface of the package to adversely influence suitability for taking out of the package; and (ii) since the pressure-sensitive adhesive layer is not exposed to the environment due to the connecting part located at the groove bottom, the components of the pressure-sensitive adhesive layer have high stability.

Under these circumstances, it is desired to develop a method of using an adhesive patch by which an adhesive patch of the type in which the liner is not separated into two or more liner pieces until just before use can be used through a simple operation.

### SUMMARY OF THE INVENTION

In view of the above, an object of the invention is to provide a method of using an adhesive patch employing a grooved liner, by which the liner can be easily peeled off.

Namely, the invention provides the following items 1 to 6.
1. A method of using an adhesive patch, in which the adhesive patch includes a backing, a pressure-sensitive adhesive layer formed on one surface of the backing, and a liner having a thickness T and laminated on the pressure-sensitive adhesive layer, wherein the liner has a groove formed from the surface opposite to the surface on which the pressure-sensitive adhesive layer is laminated and having a depth of from T/2 to less than T, and the groove has a plane shape which enables the liner to be divided into two or more liner pieces by the groove, the method comprising the steps of:
   (a) bending the adhesive patch along the groove to thereby break a liner-connecting part located at the bottom of the groove, whereby the groove gives at least a first broken part and a second broken part and the liner gives at least a first liner piece having the first broken part and a second liner piece having the second broken part; and
   (b) holding an area of the first liner piece which is near to the first broken part between fingers to remove the first liner piece from the application surface of the pressure-sensitive adhesive layer and holding an area of the second liner piece which is near to the second broken part between fingers to remove the second liner piece from the application surface of the pressure-sensitive adhesive layer, thereby exposing the application surface of the pressure-sensitive adhesive layer.
2. The method according to item 1, wherein an adhesive patch main body which is a laminate of the backing and the pressure-sensitive adhesive layer is more flexible than the liner.
3. The method according to item 1 or 2, wherein the step (a) comprises bending the adhesive patch while the surface of the first liner piece which is opposite to the surface on which the pressure-sensitive adhesive layer is laminated is kept substantially planar and the surface of the second liner piece which is opposite to the surface on which the pressure-sensitive adhesive layer is laminated is kept substantially planar.
4. The method according to any one of items 1 to 3, wherein the groove has a substantially U-shaped or substantially V-shaped cross-section.
5. The method according to any one of items 1 to 4, wherein the liner has a bending resistance of 50 mm or higher.
6. An adhesive patch to be used in the method according to any one of items 1 to 5.

In the invention, by bending an adhesive patch along a groove formed in the liner to thereby break the liner-connecting part located at the bottom of the groove, the groove is caused to give a first broken part and a second broken part.

Owing to such a simple operation in which the adhesive patch is bent along the groove, the connecting part at the bottom of the groove is broken and the broken-parts can be formed therefrom. Since areas near to the broken-parts and, in particular, the broken part function as pinching areas in the course of peeling off and removing the liner, the liner can be easily peeled off and removed. On the other hand, since the pressure-sensitive adhesive layer is prevented from being exposed to the surrounding environment until just before use owing to the connecting part, the components of the pressure-sensitive adhesive layer can have improved long-term stability. Consequently, according to the method of the invention, the liner can be easily peeled off and removed just before use to expose the application surface of the pressure-sensitive adhesive layer, while maintaining the quality of the adhesive patch until use of the patch. Namely, a high-quality patch can be easily used.

Furthermore, since the bottom of the liner in this adhesive patch is continuous until just before use, a component of the pressure-sensitive adhesive layer is inhibited from oozing out through the groove. Consequently, even when the adhesive patch which has not been used is packed in a package, this adhesive patch is less apt to adhere to the inner surface of the package and can be easily taken out of the package just before use and used.

### BRIEF DESCRIPTION OF THE DRAWINGS

Figs. 1A and 1B are slant views of examples of the adhesive patch in the method of using an adhesive patch of the invention.
Fig. 2 is a sectional view of an example of the adhesive patch in the method of using an adhesive patch of the invention.
Fig. 3 is a sectional view of the example of the adhesive patch in the method of using an adhesive patch of the invention.
Fig. 4 is a sectional view of the example of the adhesive patch in the method of using an adhesive patch of the invention.
Fig. 5 is a sectional view of the example of the adhesive patch in the method of using an adhesive patch of the invention.
Fig. 6 is a diagrammatic plan view of a test piece.
Fig. 7 is a sectional view of an example of the adhesive patch in the method of using an adhesive patch of the invention.
Figs. 8A to 8C are sectional views of examples of the adhesive patch in the method of using an adhesive patch of the invention.

### Description of Symbols

- 1: adhesive patch
- 2: backing
- 3: pressure-sensitive adhesive layer
- 4: liner
- 4a: first liner piece
- 4b: second liner piece
- 5a: first broken part
- 5b: second broken part
- 6: bending angle
- 7: sample piece
- 8: small piece of cellophane tape
- T: thickness of liner
- W: width of groove top
- Z: width of groove bottom

### DETAILED DESCRIPTION OF THE INVENTION

The invention will be explained below by reference to the accompanying drawings.

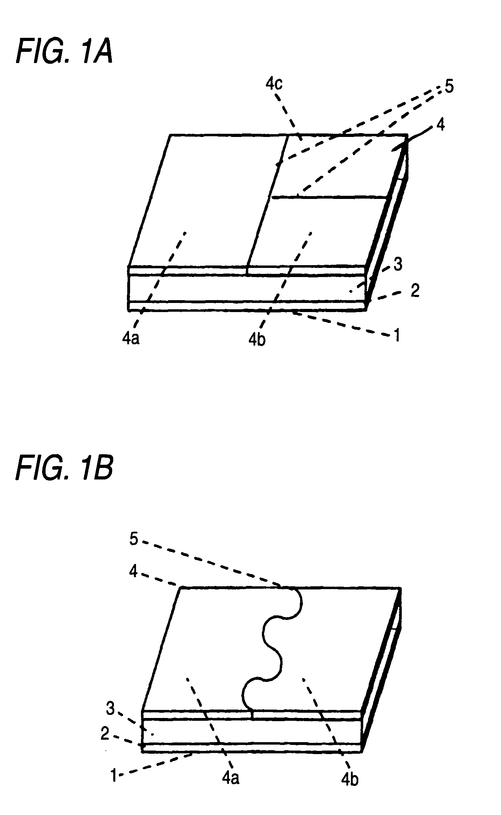

Figs. 1A and 1B are slant views of examples of the adhesive patch 1 in the method of using an adhesive patch of the invention. These adhesive patches 1 each include at least a backing 2, a pressure-sensitive adhesive layer 3 formed on one surface of the backing, and a liner 4 having a thickness T and laminated on the pressure-sensitive adhesive layer. The liner 4 has a groove 5 formed from the surface opposite to the surface on which the pressure-sensitive adhesive layer 3 is laminated and having a depth of from T/2 to less than T.

The groove 5 has a plane shape which enables the liner to be divided into two or more liner pieces by the groove 5. For example, in Fig. 1A, the liner is divided into three liner pieces 4a, 4b, and 4c by the grooves 5. In Fig. 1B, the liner is divided into two liner pieces 4a and 4b by the groove 5.

The method of using an adhesive patch according to the invention includes the following steps:
(a) bending the adhesive patch along the groove to thereby break a liner-connecting part located at the bottom of the groove, whereby the groove gives at least a first broken part and a second broken part and the liner gives at least a first liner piece having the first broken part and a second liner piece having the second broken part;
(b) holding an area of the first liner piece which is near to the first broken part between fingers to remove the first liner piece from the application surface of the pressure-sensitive adhesive layer and holding an area of the second liner piece which is near to the second broken part between fingers to remove the second liner piece from the application surface of the pressure-sensitive adhesive layer, thereby exposing the application surface of the pressure-sensitive adhesive layer; and
(c) optionally applying the application surface of the pressure-sensitive adhesive layer to a given skin part.

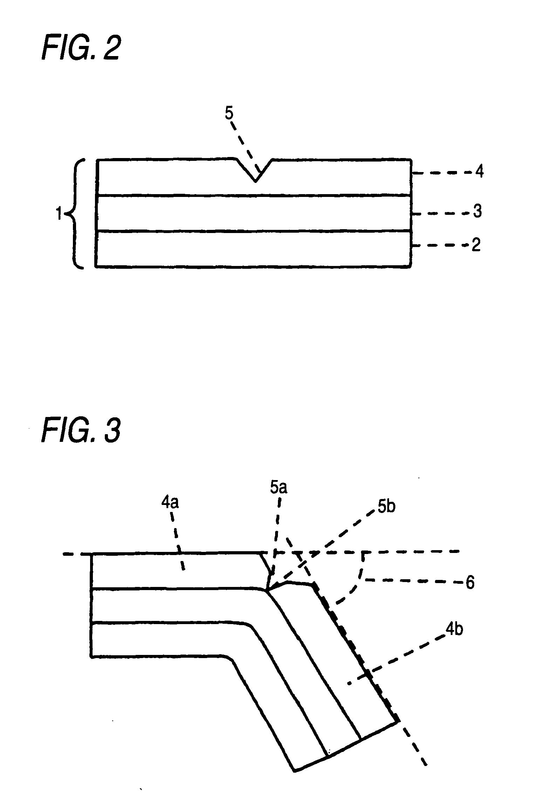

First, step (a) is explained below. Fig. 2 is a sectional view of an example of the adhesive patches shown in Figs. 1A and 1B. This adhesive patch is constituted of a pressure-sensitive adhesive layer 3, a backing 2 laminated on one surface of the pressure-sensitive adhesive layer 3, and a liner 4 laminated on the other surface of the pressure-sensitive adhesive layer 3. The liner 4 has a groove 5 such as those described above. A user holds the adhesive patch by using one of the hands to hold any desired part of the patch. The part to be held is not particularly limited. However, from the standpoint of smooth operation, it is preferred to hold part other than the liner piece to be removed first in the subsequent step (b).

Fig. 3 is a sectional view of the adhesive patch shown in Fig. 2, which has been bent along the groove 5. The liner-connecting part at the bottom of the groove 5 in Fig. 2 is broken and the groove gives a first broken part 5a and a second broken part 5b. The liner 4 gives a first liner piece 4a having the first broken part 5a and a second liner piece 4b having the second broken part 5b.

It is preferred to bend the adhesive patch 1 while the surface of the first liner piece 4a which is opposite to the surface on which the pressure-sensitive adhesive layer is laminated is kept substantially planar and the surface of the second liner piece 4b which is opposite to the surface on which the pressure-sensitive adhesive layer is laminated is also kept substantially planar, as shown in Fig. 3. By bending the adhesive patch in this manner, a stress can be concentrated on the connecting part located at the bottom of the groove 5 of the liner 4 shown in Fig. 2, whereby the connecting part can be efficiently broken. Furthermore, after the breakage, since the broken-parts formed by the breakage smoothly lift up from the application surface of the pressure-sensitive adhesive layer, pinching areas for peeling off the liner can be easily obtained. Those surfaces of the first and second liner pieces can be kept substantially planar, for example, by regulating how to apply a force with the fingers in preparation for use of the adhesive patch or by regulating the rigidity (flexibility) of the liner. The rigidity (flexibility) of the liner will be described later.

Reference is made to Fig. 3 again. It is preferred that one bending operation result in breakage of the liner-connecting part located at the bottom of the groove to give the broken-parts 5a and 5b and the liner pieces 4a and 4b. In the case where one bending operation does not result in the formation of the broken-parts and the liner pieces, the bent adhesive patch can be returned to the original shape and then bent again. This operation can be repeated according to the necessity.

The angle at which the adhesive patch is to be bent may be any desired angle which is larger than 0 degree and smaller than 180 degrees. However, from the standpoint of easily breaking the groove-bottom connecting part, the bending angle is desirably 150 degrees or smaller, preferably 120 degrees or smaller, more preferably 90 degrees or smaller, even more preferably 60 degrees or smaller, and most preferably 30 degrees or smaller.

The term "bending angle" used herein is defined as the angle which is formed by the plane including the surface of the first liner piece which is opposite to the surface on which the pressure-sensitive adhesive layer is laminated and the plane including the surface of the second liner piece which is opposite to the surface on which the pressure-sensitive adhesive layer is laminated; this angle is the angle measured from the surface of the first liner piece or from the surface of the second liner piece and is indicated by numeral 6 in Fig. 3.

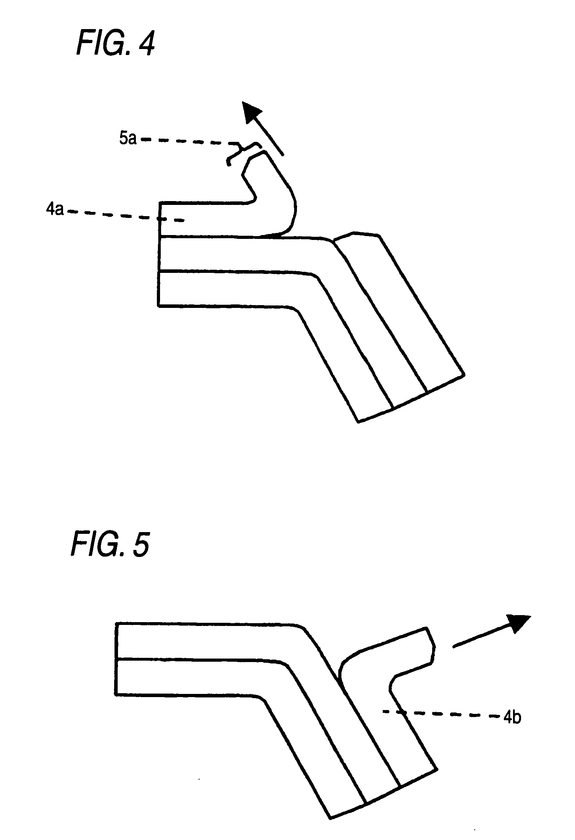

Step (b) is then explained with reference to Fig. 4. The user holds the adhesive patch by using one of the hands to hold part thereof other than the first liner piece 4a of the adhesive patch, and uses the other hand to hold the first liner piece 4a This first liner piece 4a is pulled, for example, in the direction indicated by the arrow to peel off and remove this piece from the application surface of the pressure-sensitive adhesive layer. Since the liner piece 4a is easily caught by the other hand, it is preferred to peel off and remove the first liner piece 4a while holding that area of the first liner piece 4a which is near to the first broken part 5a with the other hand, in particular, while holding the first broken part 5a with the other hand.

Reference is made to Fig. 5 next. The user likewise peels off and remove the second liner piece 4b from the application surface of the pressure-sensitive adhesive layer. Thus, the application surface of the pressure-sensitive adhesive layer is exposed.

In the subsequent step (c), which is not shown, the exposed application surface of the pressure-sensitive adhesive layer is applied to a given skin part to which the adhesive patch is to be applied, e.g., a given skin surface of an object such as a human being.

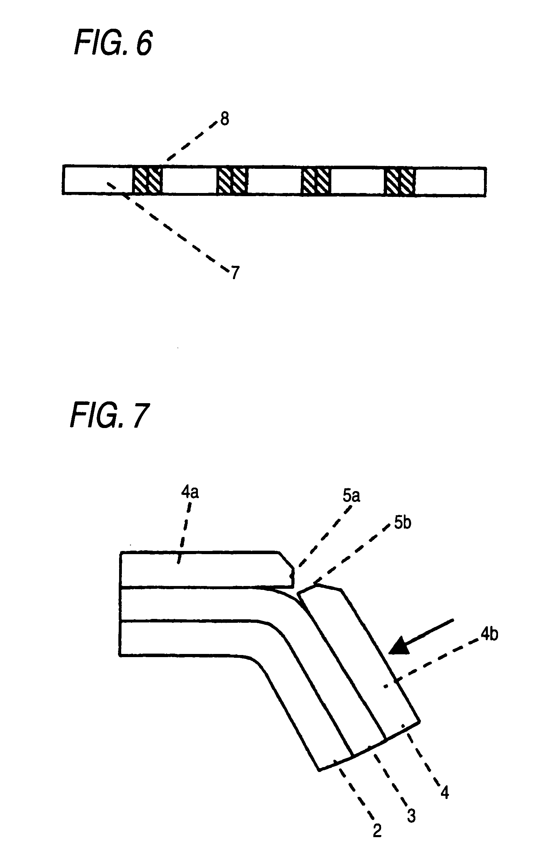

The adhesive patch from which the liner has been removed, i.e., the laminate constituted of a backing and a pressure-sensitive adhesive layer laminated on one surface of the backing, is hereinafter referred to as "adhesive patch main body". The properties of the liner and adhesive patch main body are not particularly limited. It is, however, preferred that the adhesive patch main body be more flexible than the liner. The term "flexible" herein means to have a low bending resistance. The term "bending resistance" herein means the value measured in accordance with the Japanese Industrial Standard (JIS) L1085 5.7 Bending Resistance, Method A (45° cantilever method), provided that test pieces are prepared in the following manner. In the case where the liner is a sample, the sample is cut into sample pieces each having a rectangular shape having a shorter-side length of 10 mm and a longer-side length of 30 mm. On a flat surface, five sample pieces are arranged in a row so that the longer sides of the sample pieces are located on one straight line. With respect to each adjacent two of the sample pieces, the opposed shorter sides are brought into close contact with each other and a small piece of a cellophane tape (Cello Tape (registered trademark) CT405AP, manufactured by Nichiban Co., Ltd.) cut into a square shape having a side length of 10 mm is applied to one side of contact parts of the sample pieces so that the two adjacent sample pieces come to have the same area of adhesion to the cellophane tape piece. Thus, a rectangular test piece having a shorter-side length of 10 mm and a longer-side length of 150 mm is obtained. In the case where the adhesive patch main body is a sample, a test piece is obtained in the same manner as in the case where the liner is a sample, except that cellophane tape pieces are applied to the surface of the backing. In this case, however, the test piece is examined, with the pressure-sensitive adhesive layer facing upward. Fig. 6 shows a plan view of the test pieces. Numeral 7 denotes a sample piece and 8 denotes a small piece of the cellophane tape.

Fig. 7 illustrates the case where the adhesive patch main body is more flexible than the liner. Since the adhesive patch main body, which is a laminate of a backing 2 and a pressure-sensitive adhesive layer 3, is more flexible than the liner 4, the adhesive patch main body can curve more greatly than the liner 4 when the adhesive patch 1 is bent in step (a) as shown in Fig. 7. As a result, the first broken part 5a and second broken part 5b, which are derived from the liner 4, lift up from the application surface of the pressure-sensitive adhesive layer. Consequently, pinching areas for peeling off and removing the liner piece 4a and liner piece 4b in step (b) can be obtained exceedingly easily.

The desired flexibility of the liner and adhesive patch main body is attained by selecting a material or constitution thereof, for example, a thickness thereof. Such selections are within the range of techniques employed by persons skilled in the art.

Since the pressure-sensitive adhesive layer itself is flexible, the flexibility of the adhesive patch main body is influenced by the flexibility of the backing. The material and constitution of the backing are not particularly limited. Examples thereof include various plastic films, nonwoven fabrics, paper, woven fabrics, knitted fabrics, metal foils, and laminates of these. According to need, a metal such as aluminum may be vapor-deposited on these materials.

The plastic films are not particularly limited. Examples thereof include various films made of poly(vinyl chloride) alone, copolymers of a monomer such as ethylene, propylene, vinyl acetate, acrylic acid, an acrylic ester, methacrylic acid, a methacrylic ester, acrylonitrile, styrene, or vinylidene chloride and one or more other monomers, olefin polymers such as polyethylene, polypropylene, and ethylene/vinyl acetate copolymers, polyester polymers such as poly(ethylene terephthalate) and polyether polyesters, and polyamide polymers such as polyether/polyamide block polymers.

The material and constitution of the liner are not particularly limited. Examples thereof include plastic films such as polyester films, in particular, poly(ethylene terephthalate) films, and polyolefin films and laminates of these films. Of these, polyester films, in particular, poly(ethylene terephthalate) films, are preferred because there are many kinds of polyester films having thicknesses suitable for adhesive patches and because it is easy to select a material for attaining the desired bending resistance described above. When processability and processing accuracy are taken into account, it is preferred to employ a film having an even thickness. Although such a thickness is not particularly limited, it is generally 25-200 µm, preferably 50-150 µm, from the standpoints of ease of adhesive patch production, liner cost, portability and handleability of the adhesive patch, etc. Preferably, the surface of the liner which is to face the pressure-sensitive adhesive layer is subjected to a releasant treatment in order to enable the liner to be more easily peeled off from the pressure-sensitive adhesive layer.

In order for the groove-bottom connecting part of the liner to be easily broken, the liner preferably is rigid. From this standpoint, the liner has a bending resistance of preferably 50 mm or higher, more preferably 70 mm or higher, and most preferably 100 mm or higher. There is no particular upper limit on the bending resistance of the liner. However, the bending resistance thereof is preferably 130 mm or lower because too high rigidity tends to result in reduced handleability of the adhesive patch in use.

As stated above, it is preferred that the adhesive patch main body, which is a laminate of a backing and a pressure-sensitive adhesive layer, be more flexible than the liner. In other words, it is preferred that the liner be more rigid than the adhesive patch main body, which is a laminate of a backing and a pressure-sensitive adhesive layer. From this standpoint, the bending resistance of the adhesive patch main body, which is a laminate of a backing and a pressure-sensitive adhesive layer, is preferably 5-45 mm, and more preferably 10-40 mm.

The pressure-sensitive adhesive to be used for forming the pressure-sensitive adhesive layer is not particularly limited. Examples thereof include acrylic pressure-sensitive adhesives containing an acrylic polymer; rubber pressure-sensitive adhesives such as styrene/diene/styrene block copolymers (e.g., styrene/isoprene/styrene block copolymers and styrene/butadiene/styrene block copolymers), polyisoprene, polyisobutylene, and polybutadiene; silicone pressure-sensitive adhesives such as silicone rubbers, dimethylsiloxane-based polymers, and diphenylsiloxane-based polymers; vinyl ether pressure-sensitive adhesives such as poly(vinyl methyl ether), poly(vinyl ethyl ether), and poly(vinyl isobutyl ether); vinyl ester pressure-sensitive adhesives such as vinyl acetate/ethylene copolymers; and polyester pressure-sensitive adhesives produced from a carboxylic acid ingredient such as dimethyl terephthalate, dimethyl isophthalate, or dimethyl phthalate and a polyhydric alcohol ingredient such as ethylene glycol.

Of those pressure-sensitive adhesives, acrylic pressure-sensitive adhesives are preferred. This is because acrylic pressure-sensitive adhesives can be easily crosslinked and can give a pressure-sensitive adhesive layer capable of holding a large amount of a liquid ingredient therein and giving a soft feeling during wear on the skin. In the case where the pressure-sensitive adhesive layer contains a drug, rubber pressure-sensitive adhesives are preferred from the standpoint of drug stability.

According to need, a drug can be incorporated in the pressure-sensitive adhesive layer in the adhesive patch of the invention. The drug herein is not particularly limited. Preferred is a drug which can be administered to a mammal such as a human being through the skin, i.e., which is percutaneously absorbable. Selections of such drugs are within the range of techniques employed by persons skilled in the art.

An organic liquid ingredient may be incorporated in the pressure-sensitive adhesive layer for the purpose of, e.g., regulating pressure-sensitive adhesive properties or accelerating the percutaneous absorption of a drug. The organic liquid ingredient is not particularly limited. Examples thereof include fatty acid alkyl esters produced from a higher fatty acid having 12-16 carbon atoms and a lower monohydric alcohol having 1-4 carbon atoms. Examples of the higher fatty acid having 12-16 carbon atoms include lauric acid, myristic acid, and palmitic acid. Examples of the lower monohydric alcohol having 1-4 carbon atoms include methyl alcohol, ethyl alcohol, propyl alcohol, and isopropyl alcohol.

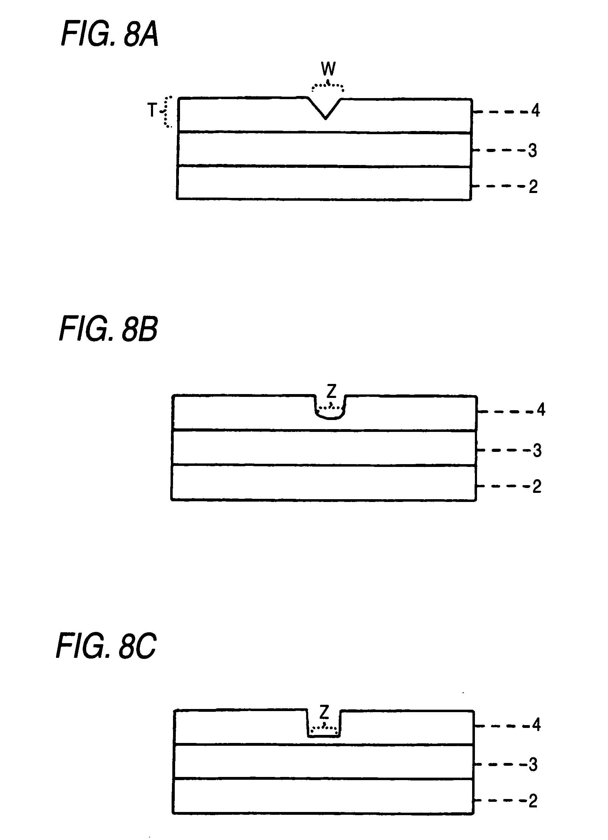

In Figs. 8A to 8C are shown diagrammatic sectional views of some embodiments of the adhesive patch. In each of these views, the liner 4 has a thickness T. As described above, the liner 4 in the invention has a groove 5 formed from the surface opposite to the surface on which the pressure-sensitive adhesive layer is laminated and having a depth of from T/2 to less than T. In case where the depth of the groove is smaller than T/2, there is a possibility that when the adhesive patch is used, the groove-bottom connecting part of the liner cannot be easily broken and, hence, liner pieces cannot be easily peeled off. From this standpoint, the depth of the groove is preferably 3T/4 or larger, more preferably 7T/8 or larger.

The depth of the groove must be smaller than T. In case where the depth of the groove is T or larger, this liner before use is in the state of having been separated into two or more liner pieces and does not have a groove-bottom connecting part. Such a groove forms a so-called "slit" in the liner. There are cases where this liner impairs the suitability of the adhesive patch for taking out of a package and reduces the stability of a component of the pressure-sensitive adhesive layer.

The cross-sectional shape of the groove 5 is not particularly limited. Examples thereof include the substantially V-shaped cross-section shown in Fig. 8A, the substantially U-shaped one shown in Fig. 8B, and the substantially rectangular one shown in Fig. 8C. These shapes include those in which the sides or angles thereof are partly curved or deformed. From the standpoint of efficiently breaking the liner-connecting part located at the groove bottom, the groove preferably has a substantially V-shaped or substantially U-shaped cross-section.

With regard to the substantially V-shaped cross-section, it is preferable that the tip has a cross-sectional shape including an acute angle such as the cross-sectional shape shown in Fig. 8A, from the standpoint of enabling the groove-bottom connecting part of the liner to be easily broken.

The width W of the top of the groove 5 is not particularly limited. However, the width of the top of the groove 5 is preferably 300 µm or smaller, and more preferably 250 µm or smaller, regardless of the cross-sectional shape of the groove 5. When the width W of the top of the groove 5 is regulated to 300 µm or smaller, the liner is better to the touch. The smaller the width W of the top of the groove, the more the liner is preferred from the standpoint of touch. However, the width W is preferably 1 µm or larger from the standpoint of groove visibility. The term "width of the top of the groove" as used herein means the width of the groove as measured on the liner surface, and is expressed by W in the example shown in Fig. 8A.

The width Z of the bottom of the groove 5 is not particularly limited so long as it exceeds 0 µm. However, the width Z is preferably 200 µm or smaller, and more preferably 100 µm or smaller, from the standpoint of inhibiting the groove-bottom connecting part of the liner from breaking until just before use.

In Fig. 8B, the groove 5 has a substantially U-shaped cross-section and the bottom of this groove 5 has a substantially curved surface. In Fig. 8C, the groove 5 has a substantially rectangular cross-sectional shape and the bottom of this groove has a substantially flat surface. In these cases, the width Z of the bottom of the groove is not particularly limited so long as it exceeds 0 µm. However, the width Z is preferably 200 µm or smaller, and more preferably 100 µm or smaller, from the standpoint of inhibiting the groove-bottom connecting part of the liner from breaking until just before use.

The term "width of the bottom of the groove" herein means the width of the groove as measured at the deepest part thereof, and is expressed by Z in Fig. 8B and Fig. 8C. In the case of a groove which does not have a deepest-part region having a substantially flat or substantially curved surface, the width of the bottom of this groove is substantially 0 µm.

The values used herein for indicating cross-sectional shapes including groove width and groove depth mean values measured with a microscope (manufactured by Keyence Corp.). The term connecting-part thickness means a value calculated by subtracting the groove depth from the liner thickness.

As stated above, the groove has a plane shape which enables the liner 2 to be divided into two or more liner pieces. There are various kinds of such plane groove shapes. Examples thereof include those which extend from a first position in an edge of the liner to a second position in another edge of the liner. It is preferred that the groove have a plane shape in which the first position differs from the second position, from the standpoints of enabling efficient liner division and easy liner removal and enabling the adhesive patch to have satisfactory handleability. More specifically, in the case where the liner has a substantially rectangular shape, examples of the plane shape of the groove include a substantially straight line and a curve such as wavy line, which extend from a certain position in one side of the liner, in particular from an approximate center of the side, to a certain position in the side which is opposed to that side, in particular to an approximate center of this side. From the standpoint of easy production, a substantially straight line is preferred. Use of a wavy line has an advantage that immediately after liner division, wave crest parts of the liner lift up from the pressure-sensitive adhesive layer and these parts can be used as a pinching area to easily peel off the liner.

The adhesive patch and adhesive preparation described above are produced, for example, in the following manner. A liner is prepared, and a pressure-sensitive adhesive layer is laminated on one surface of the liner. A backing is then laminated on the pressure-sensitive adhesive layer. Alternatively, a backing is prepared, and a pressure-sensitive adhesive layer is laminated on one surface of the backing. A liner is then laminated on the pressure-sensitive adhesive layer. Techniques for the lamination are not particularly limited. Examples thereof include coating, adhesion, melt bonding, and fusion bonding.

In the case where the adhesive patch is an adhesive preparation, i.e., in the case where a drug is incorporated into a pressure-sensitive adhesive layer, examples of methods for drug incorporation include the mixing of a pressure-sensitive adhesive with a drug and the application and infiltration of a drug to the surface of a pressure-sensitive adhesive layer.

A groove having a depth of from T/2 to less than T is formed in a liner surface before, during and/or after liner lamination. Symbol T is the thickness of the liner. Examples of groove-forming techniques include processing with a die roll, processing with a cutter such as a razor blade, and laser processing. For forming a groove having a substantially U-shaped cross-section, laser processing is suitable. Examples of the laser processing include techniques employing a CO₂ laser or YAG laser. Conditions for the laser processing vary depending on the material and thickness of the liner to be processed. A groove having a desired cross-sectional shape can be easily formed by regulating laser output or adhesive patch feed rate (or laser beam scanning speed). For forming a groove having a substantially V-shaped cross-section, processing with a razor blade is suitable. For forming a groove having a substantially rectangular cross-section, processing with a die roll is suitable.

The adhesive patch thus obtained is usable in various applications. However, it is preferred to use it as an adhesive patch because the liner can be easily peeled off by hand.

### Examples

The invention will be explained below by reference to Examples. However, the invention should not be construed as being limited to the following Examples.

### (Production of Adhesive Patch)

An organic-solvent solution of a composition for pressure-sensitive-adhesive layer formation including polyisobutylene, a tackifier, and a drug was applied to a liner which was a poly(ethylene terephthalate) film having the bending resistance and thickness shown in Table 1, in a thickness of 75 µm on a dry basis. The composition applied was dried to remove the organic solvent. Thus, a pressure-sensitive adhesive layer was formed on the liner. The surface of this pressure-sensitive adhesive layer was applied to a backing which was a poly(ethylene terephthalate) film having the thickness shown in Table 1 to obtain an adhesive patch. Thereafter, a groove having the depth shown in Table 1 and having a U-shaped cross-section was formed in the liner surface by laser processing.

### (Experimentation)

The adhesive patch was bent by hand along the groove in the liner so that the groove was turned up. The connecting part at the groove was examined as to whether it was broken or not. The results obtained are shown in the following Table 1.

### (Evaluation Criteria)

A: the connecting part was broken by one bending operation.
B: the connecting part was broken by two or three bending operations.
C: the connecting part was not broken.

**Table 1**

| | **Bending resistance (mm)/thickness (µm) of liner** | **Bending resistance of adhesive patch main body (mm)** | **Thickness of backing (µm)** | **Groove depth (relative value, with liner thickness being T)** | **Degree of breakage of connecting part upon bending** |
|---|---|---|---|---|---|
| **Example 1** | 120/75 | 30 | 15 | T/2 | B |
| **Example 2** | 120/75 | 150 or more | 100 | 3T/4 | B |
| **Example 3** | 50/25 | 30 | 15 | 3T/4 | A |
| **Example 4** | 80/50 | 30 | 15 | 3T/4 | A |
| **Example 5** | 120/75 | 30 | 15 | 3T/4 | A |
| **Example 6** | 150/100 | 30 | 15 | 3T/4 | A*¹ |
| **Comparative Example 1** | 120/75 | 30 | 15 | T/4 | C |

| | | | | | |
|---|---|---|---|---|---|
| *¹: The adhesive patch had slightly poor handleability because the liner was rigid. | | | | | |

As apparent from Table 1, in each of the adhesive patches having a groove depth regulated to T/2 or larger, the groove-bottom connecting part of the liner was able to be easily broken by merely bending the adhesive patch along the groove and the liner was able to be easily peeled from the adhesive patch main body. In particular, in Examples 2 to 6, in which the groove depth was 3T/4 or larger, connecting-part breakage tended to be easier than in Example 1, in which the groove depth was T/2. In Examples 3 to 6, in each of which the bending resistance of the liner was higher than the bending resistance of the adhesive patch main body, connecting-part breakage tended to be easier than in Example 2, in which the bending resistance of the liner was lower than the bending resistance of the adhesive patch main body.

While the present invention has been described in detail and with reference to specific embodiments thereof, it will be apparent to one skilled in the art that various changes and modifications can be made therein without departing from the scope thereof.

This application is based on Japanese patent application No. 2008-003166 filed on January 10, 2008, the entire contents thereof being hereby incorporated by reference.

## Claims

1. A method of using an adhesive patch, in which the adhesive patch includes a backing, a pressure-sensitive adhesive layer formed on one surface of the backing, and a liner having a thickness T and laminated on the pressure-sensitive adhesive layer, wherein the liner has a groove formed from the surface opposite to the surface on which the pressure-sensitive adhesive layer is laminated and having a depth of from T/2 to less than T, and the groove has a plane shape which enables the liner to be divided into two or more liner pieces by the groove, the method comprising the steps of:
(a) bending the adhesive patch along the groove to thereby break a liner-connecting part located at the bottom of the groove, whereby the groove gives at least a first broken part and a second broken part and the liner gives at least a first liner piece having the first broken part and a second liner piece having the second broken part; and
(b) holding an area of the first liner piece which is near to the first broken part between fingers to remove the first liner piece from the application surface of the pressure-sensitive adhesive layer and holding an area of the second liner piece which is near to the second broken part between fingers to remove the second liner piece from the application surface of the pressure-sensitive adhesive layer, thereby exposing the application surface of the pressure-sensitive adhesive layer.

2. The method according to claim 1, wherein an adhesive patch main body which is a laminate of the backing and the presswe-sensitive adhesive layer is more flexible than the liner.

3. The method according to claim 1, wherein the step (a) comprises bending the adhesive patch while the surface of the first liner piece which is opposite to the surface on which the pressure-sensitive adhesive layer is laminated is kept substantially planar and the surface of the second liner piece which is opposite to the surface on which the pressure-sensitive adhesive layer is laminated is kept substantially planar.

4. The method according to claim 1, wherein the groove has a substantially U-shaped or substantially V-shaped cross-section.

5. The method according to claim 1, wherein the liner has a bending resistance of 50 mm or higher.

6. An adhesive patch to be used in the method according to claim 1.
